# EUROPÄISCHE PATENTSCHRIFT

(11) **EP 3 160 402 B1**
(45) Veröffentlichungstag und Bekanntmachung des Hinweises auf die Patenterteilung: **15.05.2019**
(21) Anmeldenummer: 15731580.5
(22) Anmeldetag: 24.06.2015
(51) Int. Cl.: A61F 5/01, B25J 9/00, A61H 3/00

(54) **BEINORTHESE UND ORTHESE**
LEG ORTHOSIS AND ORTHOSIS
ORTHÈSE ET ORTHÈSE DE JAMBE

(30) Priorität: 24.06.2014 DE 102014009028
(43) Veröffentlichungstag der Anmeldung: 03.05.2017
(73) Patentinhaber: Ottobock SE & Co. KGaA, 37115 Duderstadt (DE)
(72) Erfinder: TÜTTEMANN, Markus, 45731 Waltrop (DE); VOGEL, Carsten, 37115 Duderstadt (DE)
(74) Vertreter: Lins, Martina
(86) Internationale Anmeldenummer: PCT/EP2015/064275
(87) Internationale Veröffentlichungsnummer: WO 2015/197704

(56) Entgegenhaltungen:
- EP-A1- 2 153 804
- WO-A2-2013/186705

## Beschreibung

Die Erfindung betrifft eine Beinorthese und Orthese mit einer Befestigungseinrichtung zur Festlegung der Orthese oder Beinorthese an einem Rumpf eines Orthesennutzers und zumindest einer Gelenkeinrichtung, über die eine an einer Extremität des Orthesennutzers festlegbare erste Orthesenkomponente relativ zu der Befestigungseinrichtung verschwenkbar gelagert ist. Die Orthese ist insbesondere als Bein- oder Schulterorthese ausgebildet und vorgesehen, um als Exoskelett bei Rehabilitationsmaßnahmen eingesetzt zu werden.

Nach einem Schlaganfall oder neurologischen Krankheiten oder Verletzungen ist es vorteilhaft, eine frühzeitige Rehabilitation einzuleiten, um Einschränkungen in der Bewegungsfähigkeit, insbesondere auch in der Gehfähigkeit entgegenzuwirken. Es besteht eine positive Korrelation zwischen der Intensität des Trainings und dem Rehabilitationsergebnis. Es ist daher vorteilhaft, möglichst frühzeitig mit intensiven Rebabilitationsmaßnahmen anzufangen, was jedoch zum gegenwärtigen Zeitpunkt ein entsprechend geschultes Personal benötigt. Die manuelle Gangtherapie, die unterstützend auch auf einem Laufband durchgeführt werden kann, ist durch den notwendigen Therapeuten einerseits zeitlich limitiert und führt andererseits zu dementsprechend kurzen Therapieeinheiten.

Es existieren fest installierte Systeme mit Laufband, wie der Locomat® der Firma Hocoma oder der sogenannte "Haptic-Walker" des Fraunhofer-Instituts für Produktionsanlagen und Konstruktionstechnik, der als Roboter gestützter Laufsimulator ausgebildet ist und vor allem in der motorischen Gangrehabililation bei Schlaganfallpatienten und Patienten mit einer Teil-Querschnittslähmung oder einem Schädel-Hirn-Trauma eingesetzt wird.

Problematisch an den vorbekannten Einrichtungen ist der hohe operative Aufwand und die eingeschränkte Mobilität bzw. die stationäre Anordnung der Einrichtungen.

Die EP 2 163 226 A1 betrifft ein Außenskelett, das während des Skifahrens getragen werden soll. Das Außenskelett wird an dem Skistiefel, dem Unterschenkel, dem Oberschenkel sowie der Hüfte festgelegt. In Höhe der jeweiligen natürlichen Gelenke sind Gelenkeinrichtungen angeordnet. Im Hüftbereich ist ein Doppelgelenk angeordnet. Die Gelenkeinrichtungen zwischen dem Oberschenkel und dem Unterschenkel sind über eine schaltbare Kupplung verbunden. Ein Sensor detektiert die Bewegung und/oder Druck auf Teile des Skis und übermittelt entsprechende Signale an die Kupplung. Bei Vorliegen einer potentiellen Überlastung wird die Kupplung geschlossen, so dass Kräfte über das Außenskelett um das natürliche Gelenk herum geleitet werden können. Mit einem solchen Exoskelett können keine natürlichen Gangbewegungen ausgeführt werden.

Die DE 20 2013 009 698 U1 beschreibt eine Parallelkinematik für eine Orthese oder ein Exoskelett mit seitlichen Kopplungsstellen, wobei die idealisierten Gelenkachsen der menschlichen Extremität nicht koaxial zu den Gelenkachsen der Orthese oder des Exoskeletts verlaufen.

Die WO 2013/186705 A2 betrifft eine Exoskelettvorrichtung mit Antrieben, die an oberen oder unteren Gliedmaßen angelegt werden kann. Ein Träger kann an dem Nutzer festgelegt werden. Ein erster beweglicher Körper ist an dem Träger durch ein erstes Drehgelenk verbunden, das Drehgelenk weist eine erste Drehachse auf, die im Wesentlichen orthogonal zu der Frontalebene des Nutzers verläuft und im Wesentlichen durch den Mittelpunkt des Schultergelenkes oder Hüftgelenkes des Trägers verläuft. Ein zweiter beweglicher Körper ist an dem ersten Körper über ein zweites Drehgelenk befestigt, das eine zweite Drehachse aufweist, die im Wesentlichen orthogonal zu der ersten Drehachse verläuft und sich in dem Zentrum des Hüftgelenks oder Schultergelenks mit der ersten Achse schneidet. Ein dritter beweglicher Körper ist an dem Arm oder Bein festgelegt und mit dem zweiten beweglichen Körper durch ein drittes Drehgelenk verbunden, das eine dritte Drehachse aufweist, die im Wesentlichen parallel zu der Flexionsachse oder Extensionsachse des Ellbogens oder des Knies des Nutzers ausgerichtet ist. Die dritte Drehachse verläuft im Wesentlichen orthogonal zu der zweiten Achse und schneidet sich mit der zweiten Achse in dem Gelenkzentrum der Schulter oder des Hüftgelenks.

Aufgabe der vorliegenden Erfindung ist es, eine Orthese bereitzustellen, mit der bei einer erhöhten Mobilität ein korrektes Bewegungsmuster, insbesondere Gangverhalten erreicht werden kann.

Erfindungsgemäß wird diese Aufgabe durch eine Beinorthese mit den Merkmalen des Hauptanspruches gelöst, vorteilhafte Ausgestaltungen und Weiterbildungen der Erfindung sind in den Unteransprüchen, der Beschreibung sowie den Figuren offenbart.

Die Beinorthese mit einer Befestigungseinrichtung zur Festlegung der Beinorthese an einem Rumpf eines Orthesennutzers und zumindest einer Gelenkeinrichtung, über die eine an einer Extremität des Orthesennutzers festlegbare erste Orthesenkomponente relativ zu der Befestigungseinrichtung verschwenkbar gelagert ist, sieht vor, dass die Gelenkeinrichtung zumindest drei Gelenke aufweist, die jeweils zumindest eine Schwenkachse aufweisen und die jeweils zumindest einen Schwenkachsen sich in einem gemeinsamen Punkt schneiden, wobei die Schwenkachsen im gemeinsamen Schnittpunkt zumindest drei unabhängige Freiheitsgrade ausbilden. Durch die Ausgestaltung der Gelenkeinrichtung mit drei Schwenkachsen und einem gemeinsamen Schnittpunkt ist es möglich, ein Bewegungsverhalten der Orthese zu erzeugen, das einem natürlichen Gelenk in Gestalt eines Kugelgelenks entspricht. Es ist somit möglich, ein innerhalb des Körpers liegendes Kugelgelenk durch außen liegende Gelenke nachzubilden, so dass die Orthese einerseits die notwendige Stütz- und Führungsfunktion wahrnehmen kann und andererseits ein physiologisch korrektes Bewegungsverhalten durch den Orthesennutzer erreichbar ist. Durch die Ausgestaltung der Gelenkeinrichtung mit drei sich in einem Punkt schneidenden Gelenkachsen wird ein Kugelgelenk in einer Position, nämlich im Inneren des Körpers, erzeugt, in der bei einer Außenlagerung der Gelenke keine direkte mechanische Realisierung der Gelenke möglich wäre und somit die natürliche Bewegung eingeschränkt werden würde. Wird nachfolgend von der Orthese gesprochen, so beziehen sich die Aussagen sowohl auf Beinorthesen als auch auf Schulter-Arm-Orthesen oder Orthesen allgemein, werden spezielle Eigenschaften erläutert, wird gesondert auf die jeweilige Ausführungsform eingegangen. Bei drei Schwenkachsen, die sich in einem gemeinsamen Punkt schneiden, sind in dem gemeinsamen Schnittpunkt der drei Schwenkachsen der Gelenke drei rotatorische Freiheitsgrade ausgebildet, die unabhängig voneinander sind, so dass sich eine Bewegung der Orthese wie bei einer Kugelgelenkslagerung realisieren lässt. Es ist vorgesehen, dass zumindest zwei der zumindest drei Schwenkachsen nicht orthogonal zueinander ausgerichtet sind. Dadurch ist es möglich, dass die Gelenke außerhalb des Körpers neben den Extremitäten oder dem Rumpf an Stellen angeordnet werden können, die eine Tragbarkeit der Orthese ermöglicht. Bei nicht orthogonal zueinander ausgerichteten Achsen können von einem Gelenk kombinierte Bewegungen ausgeführt werden, ohne dass andere Gelenke mitbewegt werden müssen, die Orientierung der Achsen kann somit an die Bewegungen des Orthesennutzers angepasst werden, da in den seltensten Fällen eine isolierte Bewegung in genau einer Ebene um genau eine in dieser Ebene liegende Achse ausgeführt wird. Eine Kopplung der Gelenke über Kraftübertragungseinrichtungen findet bevorzugt nicht statt.

Der Gelenkeinrichtung kann zumindest ein Antrieb oder Kraftspeicher zugeordnet sein, um die Orthese in zumindest einer Ebene verschwenkbar und aktiv unterstützbar auszugestalten. Dadurch ist es vorteilhaft möglich, den Orthesennutzer beim Gehen zu unterstützen, beispielsweise bei der Flexion und Extension innerhalb der Sagittalebene, also beim Anheben und Absenken des Knies oder Anheben oder absenken des Armes oder eines anderen Teils der Extremität. Grundsätzlich ist es auch möglich, mehrere Antriebe vorzusehen, die um mehrere oder sämtliche Schwenkachsen der Gelenke wirksam sind. Neben einem motorischen Antrieb mit externer Energiequelle, beispielsweise einer Batterie oder einem Akkumulator, können auch Kraftspeicher zugeordnet sein, beispielsweise Federn oder dergleichen, um eine Bewegungsunterstützung vorzunehmen.

Die Freiheitsgrade der Schwenkachse zumindest eines der Gelenke der Gelenkeinrichtung beeinflusst vorteilhafterweise nicht die Freiheitsgrade der anderen Gelenk, so dass es zu keiner Mitkopplung von Bewegungen bei beispielsweise einer reinen Flexion oder Extension bzw. Adduktion oder Abduktion sowie Innenrotation oder Außenrotation kommt. In einer Ausgestaltung der Erfindung sind alle Freiheitsgrade voneinander entkoppelt ausgebildet, eine Variante der Erfindung sieht vor, dass nur ein Gelenk von den anderen Gelenken entkoppelt ist, während die Freiheitsgrade der anderen Gelenke einander beeinflussen können. Wird beispielsweise eine Achse angetrieben, so übt der Antrieb keine Momente auf die andere Achsen der Gelenkeinrichtung oder der Orthese aus. Die nicht angetrieben Achsen können sich gegenseitig beeinflussen, Momente um diese Achsen haben jedoch keine Auswirkung auf die angetriebene Achse.

Die Befestigungseinrichtung ist insbesondere bei der Ausgestaltung als Beinorthese vorteilhafterweise zur Festlegung der Orthese an dem Becken, dem Bauchbereich oder der Taille ausgebildet, wobei die erste Orthesenkomponente als Oberschenkelschiene oder Oberarmschien ausgebildet ist. Die Anordnung der Orthese mit der Befestigungseinrichtung an dem Becken, dem Bauchbereich oder der Taille ermöglicht es, die Befestigungseinrichtung zusätzlich in einem Rahmen oder an einem Gestell zu positionieren, so dass es möglich ist, auch den Rumpf abzustützen und zu tragen, so dass eine Gewichtsentlastung der Orthese während der Bewegung möglich ist. Der Orthesennutzer ist nicht darauf angewiesen, sein komplettes Körpergewicht beim Gehen oder der Bewegung zu tragen, vielmehr ist es möglich, über die Befestigungseinrichtung einen Teil der Gewichtskraft abzufangen.

Die Schwenkachsen der Gelenke der Gelenkeinrichtung schneiden sich bevorzugt in dem Drehpunkt des Kugelgelenks des Orthesennutzers, also im Hüftgelenk, wenn die Orthese als Beinorthese ausgebildet ist, oder in dem Schultergelenk, wenn die Orthese als Schulter-Arm-Orthese ausgebildet ist, wobei aufgrund der vorhandenen Ausgleichsmöglichkeiten bei der Fixierung der Orthese an dem Orthesennutzer, des auftretenden Spiels sowie der Variabilität des menschlichen Körpers es ausreicht, wenn die Schwenkachsen durch einen gedachten Kugelkörper verlaufen, der in der Größe dem Gelenkkopf des Hüftgelenks oder des Schultergelenks entspricht.

Die erste Orthesenkomponente kann mehrere, gelenkig miteinander verbundene Abschnitte aufweisen, wobei jeder Abschnitt durch ein Gelenk mit dem anderen Abschnitt verbunden ist. Ein Abschnitt ist mit der Befestigungseinrichtung gekoppelt, ein weiterer Abschnitt kann mit einer weiteren Orthesenkomponente gelenkig verbunden sein. Durch die gelenkige Verbindung der ersten Orthesenkomponente, die an dem Oberschenkel oder Oberarm des Orthesennutzers festgelegt ist, ist es möglich, dass vollständige Bein oder den gesamten Arm über die Orthese abzustützen, also auch den Unterschenkel und den Fuß oder den Unterarm und die Hand. Die weitere Orthesenkomponente bzw. die weiteren Orthesenkomponenten sind an der jeweiligen Extremität festlegbar ausgebildet, wobei die Schwenkachsen der Gelenke zwischen den weiteren Orthesenkomponenten bzw. zwischen der ersten Orthesenkomponente und der sich daran anschließenden Orthesenkomponente im angelegten Zustand der Beinorthese die mit den Gelenkachsen der jeweiligen Extremität, also den Gelenkachsen der natürlichen Gelenke fluchten, um durch die Orthesenkomponente keine Beeinträchtigung der natürlichen Beweglichkeit der jeweiligen Extremität zu bewirken.

Die Schwenkachsen der Gelenke der Gelenkeinrichtung können jeweils einen Freiheitsgrad aufweisen, bevorzugt jeweils genau einen Freiheitgrad, die nicht kollinear zueinander verlaufen, so dass sie durch die Ergänzung der Freiheitsgrade eine Bewegung der Extremität und damit auch der Orthese erreichen lässt, die der eines Kugelgelenkes entspricht.

Ein Freiheitsgrad einer Schwenkachse der Gelenkeinrichtung ist vorteilhafterweise in der Sagittalebene orientiert, wogegen die beiden anderen Freiheitsgrade der Schwenkachsen der Gelenke der Gelenkeinrichtung in der Frontalebene orientiert sind, um eine im Gelenkdrehpunkt vertikal projizierte Achse zu erzeugen.

Sofern Bewegungseinschränkungen notwendig sind oder der volle Bewegungsbereich der natürlichen Gelenke nicht ausgeschöpft werden soll, sind die Schwenkbereiche der Gelenke einstellbar ausgebildet, zumindest der Schwenkbereich eines Gelenkes. Dadurch wird ermöglicht, dass sich eine schrittweise Anpassung an die jeweiligen maximalen Bewegungsradien erreichen lässt, so dass medizinisch notwendigen Bewegungseinschränkungen einerseits und Therapiefortschritten andererseits Rechnung getragen werden kann.

Eine Orthese mit einer Befestigungseinrichtung zur Festlegung der Orthese an einem Rumpf eines Orthesennutzers und zumindest einer Gelenkeinrichtung, über die eine an einer Extremität des Orthesennutzers festlegbare erste Orthesenkomponente relativ zu der Befestigungseinrichtung verschwenkbar gelagert ist, wobei die Gelenkeinrichtung zumindest drei Gelenke aufweist, die jeweils eine Schwenkachse aufweisen und die Schwenkachsen sich in einem gemeinsamen Punkt schneiden, sieht vor, dass die Freiheitsgrade einer Schwenkachse zumindest eines Gelenkes der Gelenkeinrichtung die Freiheitsgrade der Schwenkachsen der übrigen Gelenke nicht beeinflussen, wodurch es möglich ist, zumindest um eine Achse einen Antrieb wirksam werden zu lassen, ohne ungewollte Bewegungen zu verursachen oder ungewollte Bewegungen bei einem natürlichen Bewegungsantrieb durch Muskeln zu einzuleiten. Die obigen Ausführungen zu Beinorthesen oder Schulter-Arm-Orthesen gelten für die Orthese entsprechend.

Die Schwenkachsen der Gelenke schneiden sich bevorzugt in einem gemeinsamen Punkt und bilden im gemeinsamen Schnittpunkt wenigstens drei unabhängige Freiheitsgrade.

Nachfolgend werden Ausführungsbeispiele der Erfindung anhand der beigefügten Figuren näher erläutert. Es zeigen:
- Figur 1 -: eine schematische Darstellung eines menschlichen Körpers mit Gelenkachsen;
- Figur 2 -: eine schematische Darstellung einer angelegten Orthese;
- Figur 3 -: eine Variante der Figur 1,
- Figur 4 -: eine Darstellung einer angelegten Orthese in der Position gemäß Figur 1;
- Figur 5 -: eine Einzeldarstellung einer Gelenkeinrichtung mit Teilen einer Befestigungseinrichtung;
- Figur 6: eine Teilansicht einer Ausführungsform der Erfindung;
- Figur 7: eine Draufsicht auf einen Orthesennutzer mit angelegter Orthese;
- Figur 8: eine Seitenansicht einer Orthese im Ausgangszustand;
- Figur 9: eine Seitenansicht der Orthese mit angehobenem rechten Bein; sowie
- Figur 10: eine Orthese in Frontalansicht in Ausgangsstellung.

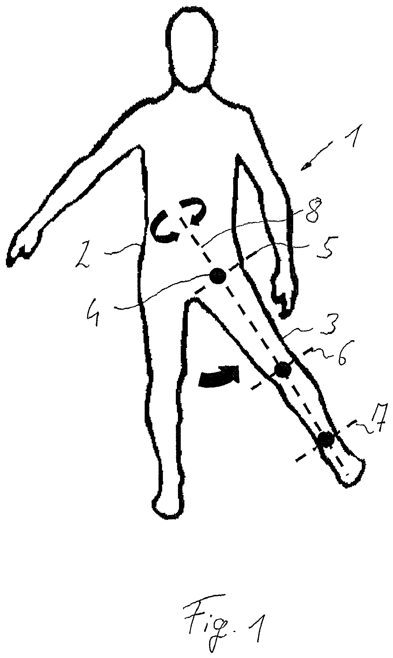

Figur 1 zeigt in einer schematischen Darstellung einen Orthesennutzer 1 mit einem Rumpf 2 und zwei unteren Extremitäten 3. Die linke Extremität 3 des Orthesennutzers 1 ist abduktiert, also in der Frontalebene nach außen verschwenkt. Das Verschwenken findet dabei um die Hüftachse 4 statt, die durch das nicht näher dargestellte Hüftgelenk verläuft. Im Bereich des Knies der unteren Extremität ist eine Knieachse 6 eingezeichnet, die eine Flexion und Extension des Unterschenkels ermöglicht, eine Knöchelachse 7 ermöglicht eine Plantarflexion und eine Dorsalextension. Darüber hinaus ist die Beinachse 8 eingezeichnet, um die eine Innenrotation oder Außenrotation der unteren Extremität 3 erfolgt, so dass der Fuß bei einem gestreckten Bein mit der Fußspitze nach außen bzw. nach innen verdreht werden kann.

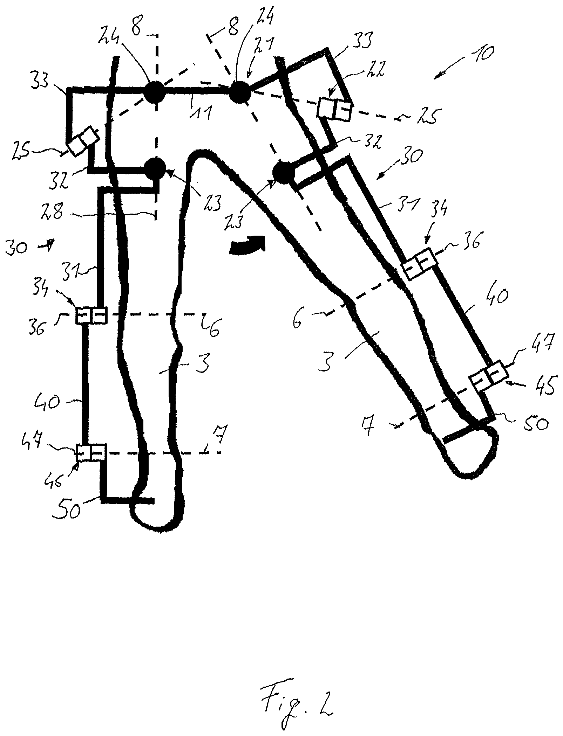

Figur 2 zeigt in einer Teildarstellung die Haltung des Orthesennutzers 1 gemäß Figur 1 mit angelegter Orthese 10, wobei die Orthese 10 schematisch dargestellt ist. Die Orthese 10 weist eine schematisch dargestellte Befestigungseinrichtung 11 auf, die zur Festlegung an dem Rumpf 2 des Orthesennutzers 1 ausgebildet ist. Die Befestigungseinrichtung 11 kann als Manschette, Schale oder ein mehrteiliger Schaft ausgebildet sein, der um den Rumpf 2, beispielsweise im Bereich der Taille, der Hüfte oder des Beckens an dem Orthesennutzer 1 festlegbar ist. Die Festlegung kann über Gurte, Schnallen, Klammern oder einstellbare Befestigungselemente erfolgen. An der Befestigungseinrichtung 11, die sich auch im Bereich des Beckens erstreckt, sind für jede Extremität 3 eine erste Orthesenkomponente 30 gelenkig befestigt. Die erste Orthesenkomponente 30 weist drei Abschnitte 31, 32. ,33 auf, die miteinander über Gelenke 22, 23 verbunden sind. Der der Befestigungseinrichtung 11 nächste Abschnitt 33, auch Befestigungsabschnitt genannt, ist ebenfalls über ein Gelenk 21 an der Befestigungseinrichtung 11 gelagert, so dass die gesamte erste Orthesenkomponente 30 über drei Gelenke 23, 22, 21 an der Befestigungseinrichtung 11 verschwenkbar gelagert ist. Die erste Orthesenkomponente 30 ist an dem Oberschenkel fixiert, vorteilhafterweise im Bereich des Oberschenkels des Orthesennutzers 1 über übliche Manschetten oder Gurte 60, die nur schematisch dargestellt sind. Distal zu der Oberschenkelschiene 31 schließt sich eine Unterschenkelschiene 40 an, die mit der Oberschenkelschiene 31 über ein Orthesenkniegelenk 34 verschwenkbar gekoppelt ist. An dem distalen Ende 40 der Unterschenkelschiene schließt sich ein Orthesenknöchelgelenk 45 an, das die Unterschenkelschiene 40 mit einer Fußschiene 50 gelenkig koppelt. Sowohl die Fußschiene 50 als auch die Unterschenkelschiene 40 sind über Befestigungsmittel, beispielsweise Gurte 60, Manschetten, Klettverschlüsse oder dergleichen an dem jeweiligen Teil der unteren Extremität 3 befestigt. Die Gelenkachsen 36, 47 des Orthesenkniegelenkes 34 bzw. des Orthesenknöchelgelenkes 45 sind kollinear zu den Gelenkachsen 6, 7 des natürlichen Kniegelenks bzw. Knöchelgelenks ausgebildet, so dass eine natürliche Bewegung der unteren Extremitäten 3 erfolgen kann. Bei dem Orthesenknöchelgelenk 45 ist auch nur eine Plantarflexion bzw. Dorsalextension vorgesehen.

Die Gelenkeinrichtung 20 mit den drei Gelenken 21, 22, 23 ist so aufgebaut, dass die Gelenkachsen 24, 25, 28 der jeweiligen Gelenke 21, 22, 23 sich in einem gemeinsamen Punkt 100 schneiden, wobei der Schnittpunkt der Gelenkachsen 24, 25, 28 dem Drehzentrum des natürlichen Hüftgelenkes entspricht. Dabei ist es nicht zwingend notwendig, dass sich die Achsen 24, 25, 28 in genau einem geometrischen Punkt treffen, es kann ausreichend sein, wenn sämtliche Achsen 24, 25, 28 durch einen Kugelbereich verlaufen, der dem Kugelkopf des Hüftgelenks entspricht.

In dem dargestellten Ausführungsbeispiel sind sämtliche Gelenkachsen 24, 25, 28, 36, 47 der Gelenke 21, 22, 23, 34, 45 der jeweiligen unilateralen Orthese mit nur genau einem rotatorischen Freiheitsgrad ausgestattet. Die Gelenkachsen 24, 25, 28 der Gelenkeinrichtung 20 der ersten Orthesenkomponente 30 schneiden sich in genau einem Punkt, der im Drehzentrum des natürlichen Hüftgelenks liegt, wobei die Drehachse 24 des ersten Gelenks 21 senkrecht zur Frontalebene verläuft, so dass eine Abduktion bzw. Adduktion möglich ist. Die Schwenkachse 25 des zweiten Gelenks 22 dient überwiegend zur Extension bzw. Flexion des Oberschenkels und stellt einen Hüftdrehpunkt dar, der zum Anheben bzw. Absenken des Kniegelenks notwendig ist. Das dritte Gelenk 23, das unterhalb des natürlichen Hüftgelenkes angeordnet ist, bildet eine Drehachse 28 aus, die im Wesentlichen kollinear zu der Beinachse 8 verläuft und somit eine Innenrotation oder Außenrotation des Fußes bzw. der gesamten unteren Extremität ermöglicht.

Die jeweiligen rotatorischen Freiheitsgrade der Schwenkachsen 24, 25, 28 der Gelenkeinrichtung 20 beeinflussen sich nicht und sind so zueinander ausgerichtet, dass die natürliche Bewegung des Hüftgelenkes über einen weiten Verschwenkbereich der unteren Extremität 3 ermöglicht wird, insbesondere ist eine Abduktion und Adduktion sowie eine Innenrotation und Außenrotation der jeweiligen unteren Extremität 3 möglich.

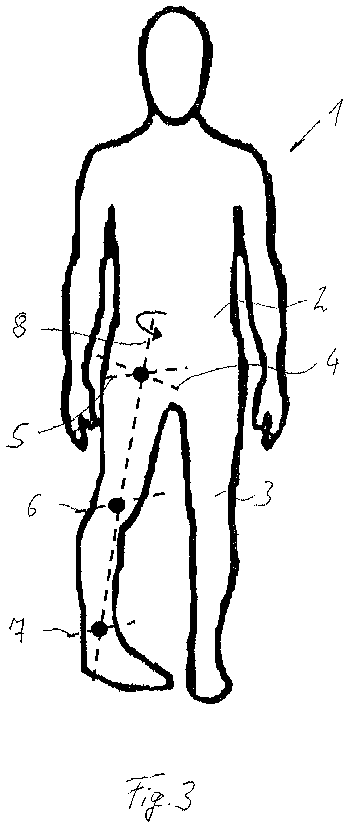

Figur 3 zeigt einen Orthesennutzer 1 ohne Orthese mit einer innenrotierten rechten Extremität 3. Die Orientierung der jeweiligen Achsen ist eingezeichnet, die Knöchelgelenksachse 7 sowie die Kniegelenksache 6 verlaufen angenähert parallel zueinander, die Beinachse 8 ermöglicht die Innenrotation und Außenrotation des Fußes, in dem Hüftgelenk schneiden sich zwei weitere Hüftachsen, nämlich die Hüftachse 4, die in der Sagittalebene liegt und eine Abduktion und Adduktion der unteren Extremität 3 ermöglicht, sowie die Hüftachse 5, die in der Frontalebene liegt und eine Extension und Flexion der unteren Extremität 3 ermöglicht.

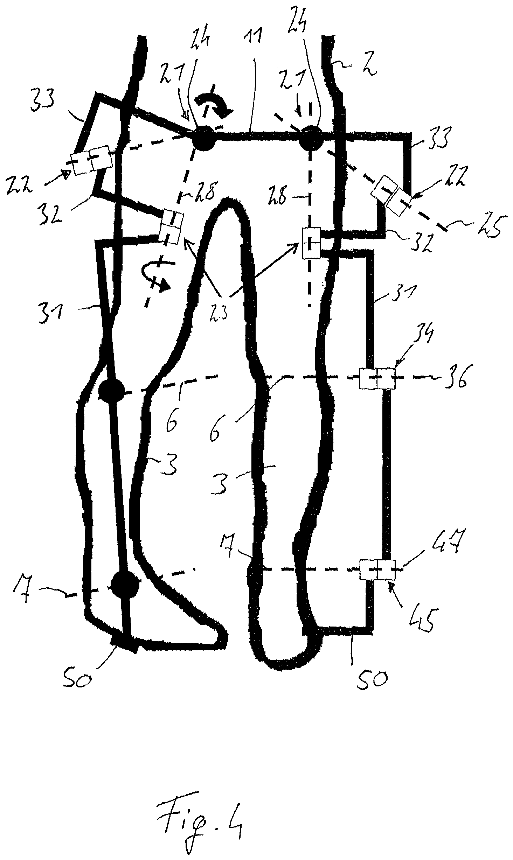

Figur 4 zeigt eine Detailansicht der Stellung gemäß Figur 3 mit angelegter Orthese 10. Die Innenrotation der rechten Extremität 3 wird überwiegend durch eine Verkippung um die Schwenkachse 24 des rechten ersten Gelenks 21 sowie durch eine Verdrehung um die Schwenkachse 28 des dritten Gelenks 23 erreicht. Durch die Überlagerung der Drehbewegungen in den beiden Gelenken 21, 23 ist es möglich, eine Innenrotation und dementsprechend auch eine Außenrotation über einen aufgesetzten Fußteil 50 zu ermöglichen, wodurch es mit der Orthese ohne weiteres möglich ist, eine Kurve zu gehen, ohne eine Zirkumduktion auszuführen, was ein unphysiologisches Gangbild zur Folge hätte. Weiterhin ist der Figur 4 zu entnehmen, dass die Schwenkachse 28 des dritten Gelenkes 23 nicht orthogonal zu den Schwenkachsen 24, 25 des ersten und zweiten Gelenks orientiert ist, sondern eine Schrägstellung zu den anderen Schwenkachsen 23, 24 aufweist. Über die schräggestellte Schwenkachse 28 des dritten Gelenkes 23 ist es möglich, eine Bewegung der unteren Extremität in zwei Ebenen auszuführen, nämlich eine Beinrotation und eine Abduktion oder Adduktion. Für den Fall, dass lediglich eine Innen- oder Außenrotation des Beines gewünscht ist, wird die zusammen damit eingeleitete Abduktion oder Adduktion in dem ausschließlich in dieser Ebene beweglichen Drehgelenk 21 kompensiert, ohne dass die in der Orthese befindliche Extremität eine Abduktion oder Adduktion ausführt beziehungsweise in eine solche Bewegung gezwungen wird. Die Orthese führt also das Bein korrekt in der Bewegung, die nicht identische Orientierung der Schwenkachsen 24, 25, 28 zu den idealen Bewegungsachsen des Beines wird innerhalb der Orthese kompensiert, wobei das zweite Drehgelenk 22 bei einer Beinrotation nicht involviert wird und von den beiden anderen Drehgelenken 21, 23 entkoppelt ist.

Das dritte Gelenk 23 ist unterhalb des natürlichen Hüftgelenkes angeordnet und so in posteriorer und distaler Richtung zu dem Hüftgelenk positioniert, dass es sich auf der Rückseite des Oberschenkels und in posteriorer Richtung beabstandet zu dem Oberschenkel befindet. Eine kollineare Ausrichtung der Schwenkachse 28 des dritten Gelenkes 23 zu der Beinachse 8, die die Innenrotation oder Außenrotation des Fußes ermöglicht, ist nicht möglich, da hierzu das dritte Drehgelenk 23 unterhalb des Fußes angeordnet sein müsste oder aber eine parallele Ausrichtung der Schwenkachse 28 beabstandet zu der Rotationsachse verlaufen würde, was verhindern würde, dass sich die Schwenkachsen 24, 25, 28 in einem gemeinsamen Punkt 100 schneiden.

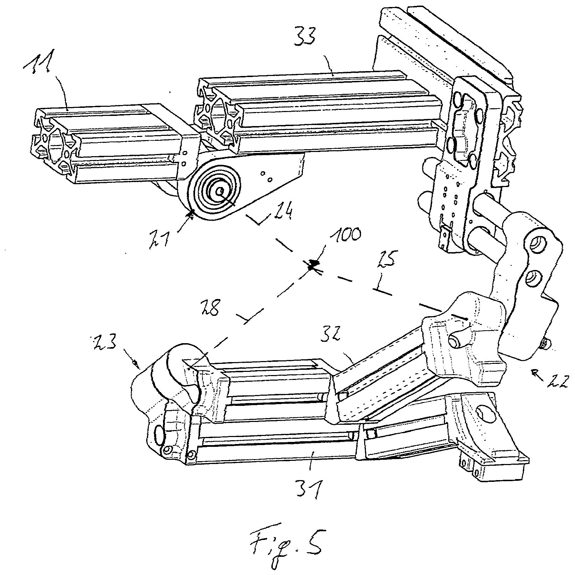

Figur 5 zeigt eine Detaildarstellung der Gelenkeinrichtung 20 mit den drei Gelenkabschnitten 31, 32, 33, die jeweils über Gelenke 22, 23 miteinander verbunden sind. Das Befestigungselement 11 ist teilweise dargestellt und stellt gleichzeitig eine Verbindung zwischen den jeweils ersten Gelenken 21 dar, deren Schwenkachsen 24 eine Abduktion und Adduktion der unteren Extremität ermöglichen.

In der Figur 5 ist zu erkennen, dass die jeweiligen Schenkachsen 24, 25, 28 der Gelenke 21, 22, 23 sich in einem gemeinsamen Punkt 100 schneiden, der innerhalb des Orthesennutzers liegt, also das virtuelle Gelenk in den Drehpunkt des natürlichen Hüftgelenkes hinein projiziert ist. Dadurch ist es möglich, eine vollständige und natürliche Beweglichkeit innerhalb der Hüfte zu bewirken. Sämtliche Gelenke 21, 22, 23 der Gelenkeinrichtung 20 weisen jeweils nur einen Freiheitsgrad auf, nämlich einen rotatorischen Freiheitsgrad, wobei die Schwenkachse 24 des ersten Gelenks 21, das die erste Orthesenkomponente 30 mit der Befestigungseinrichtung 11 verbindet, in der Sagittalebene liegt, während die beiden anderen Schwenkachsen 25, 28 im Wesentlichen in der Frontalebene liegen, wobei die Freiheitsgrade der Schwenkachsen 24, 25, 28 der Gelenke 21, 22, 23 der Gelenkeinrichtung 20 sich nicht beeinflussen, also voneinander entkoppelt sind.

In der Figur 5 ist die Ausgestaltung eines Teils einer Orthese für ein linkes Bein dargestellt. Das erste Drehgelenk 21 ist so positioniert, dass dessen Schwenkachse 24 im Wesentlichen auf der gleichen Höhe des natürlichen Hüftgelenkes positioniert ist, wohingegen das zweite Gelenk 22, das überwiegend für die Flexion und Extension zuständig ist, leicht unterhalb der Höhe des natürlichen Hüftgelenkes und somit unterhalb der waagerechten Ebene, die durch die erste Schwenkachse 24 verläuft, angeordnet ist. Das Drehgelenk 22 kann auch auf gleicher Ebene mit oder über dem natürlichen Hüftgelenk liegen. Die zweite Schwenkachse 25, die durch das zweite Drehgelenk 22 verläuft, schneidet die erste Schwenkachse 24 in dem gemeinsamen Punkt 100, die zweite Schwenkachse 25 verläuft somit nach schräg oben orientiert in Richtung auf den gemeinsamen Punkt 100.

Die dritte Schwenkachse 28 des dritten Gelenkes 23 liegt bevorzugt innerhalb einer Sagittalebene, die durch die erste Schwenkachse 24 verläuft. Der Winkel zwischen den beiden Achsen 24, 28 ist jedoch auch bei einem Verlauf außerhalb einer solchen Sagittalebene ein spitzer Winkel, so dass es möglich ist, das dritte Gelenk 23 unterhalb und hinter dem Hüftgelenk und unterhalb des Beckens des Orthesennutzers zu positionieren.

Aufgrund der Positionierung des zweiten Drehgelenks 22 unterhalb der waagerechten Ebene, die durch den gemeinsamen Punkt 100 verläuft, ergibt sich ein ebenfalls spitzer Winkel zwischen den Schwenkachsen 25, 28 des zweiten und dritten Gelenks 22, 23. Alle drei Achsen 24, 25, 28 stehen somit nicht orthogonal aufeinander.

In allen Gelenken 21, 22, 23, 34, 45 können Schwenkbereichsanschläge einstellbar ausgebildet sein, um bei physiologischen Einschränkungen oder notwendigen therapeutischen Einschränkungen eine Begrenzung oder Anpassung der maximalen Schwenkwinkel vornehmen zu können.

Darüber hinaus ist es möglich, dass zumindest einer Gelenkeinrichtung ein Antrieb oder ein Kraftspeicher zugeordnet ist, beispielsweise einen Motor, um den Orthesennutzer bei der Bewegung zu unterstützen, beispielsweise um eine Extension oder Flexion des Oberschenkels und/oder des Unterschenkels zu bewirken.

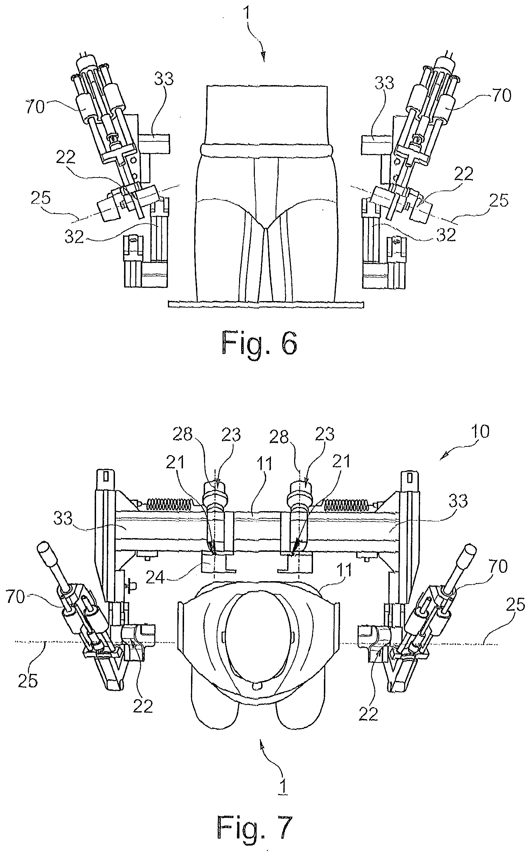

In der Figur 6 ist eine Teilansicht der Orthese an einem Orthesennutzer 1 in Frontalansicht dargestellt. Neben dem Befestigungsabschnitt 33 sowie ein Gelenk 22, dessen Schwenkachse 25 im Wesentlichen eine Flexion und Extension in der Hüfte ermöglicht und den Befestigungsabschnitt 3 mit dem nachfolgenden Abschnitt 32 verbindet, ist beiderseits des Orthesennutzers 1 ein Antrieb 70 vorgesehen, über den die Gelenkeinrichtung 30 mit der an dem Oberschenkel befestigten Orthese um die Schwenkachse 25 herum bewegt werden kann, um eine Flexion oder Extension im Hüftgelenk für den Patienten zu erleichtern.

Figur 7 zeigt eine Draufsicht auf eine an einen Orthesennutzer 1 angelegte Orthese 10, die Befestigungsmittel in Gestalt einer Rückmanschette sind an dem Orthesennutzer 1 über einen Bauchgurt festgelegt. In der Figur ist zu erkennen, dass die Schwenkachse 25 des seitlich neben dem Orthesennutzer 1 angeordneten Gelenks 25 in der Ausgangsstellung im Wesentlichen in der Frontalebene verläuft und durch das natürliche Hüftgelenk geht, so dass die Antriebe 70, die mit den Gelenkabschnitten 33, 32 gekoppelt sind, die Flexion sowohl der rechten als auch der linken unteren Extremität unterstützen oder bewirken kann. In der Draufsicht ist ebenso zu erkennen, dass das Gelenk 21, das den Befestigungsabschnitt 32 mit der Befestigungseinrichtung 11 verbindet, hinter dem Orthesennutzer angeordnet ist und die Schwenkachse 24 im Wesentlichen in der Sagittalebene waagerecht nach vorne zeigt, um eine Abduktion oder Adduktion der unteren Extremität zu ermöglichen. Dazu wird der jeweils äußere Abschnitt des Verbindungsabschnittes 33 angehoben bzw. abgesenkt.

In der Draufsicht der Figur 7 ist ebenso zu erkennen, dass das dritte Gelenk 23, das distal sowohl zu dem ersten Gelenk 21 als auch zu dem zweiten Gelenk 22 angeordnet ist, hinter dem Orthesennutzer 1 positioniert ist und eine schräg nach oben weisende Schwenkachse 28 ausbildet, die ebenfalls im Wesentlichen in der Sagittalebene der jeweiligen Extremität liegt und sich mit den beiden anderen Drehachsen 24, 25 der beiden anderen Gelenke 21, 22 in dem Bereich des natürlichen Hüftgelenkes schneidet.

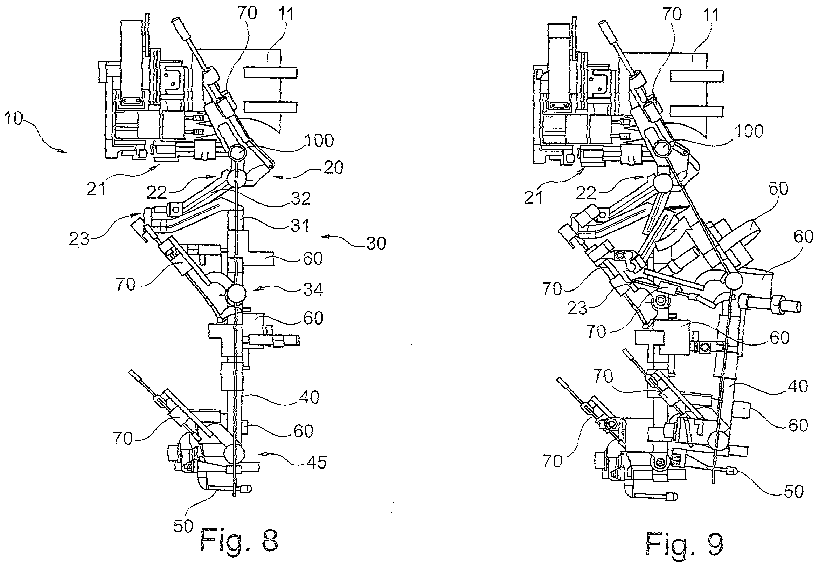

Figur 8 zeigt in einer Seitenansicht die Orthese 10 in einem Ausgangszustand. Die Befestigungseinrichtung 11 mit der daran befestigten Orthese 10 ist in Gestalt einer Manschette, die über Klettverschlüsse in Gestalt von Gurten an dem Rumpf festgelegt werden können, ausgebildet. Ebenso sind die Oberschenkelschiene 31 der ersten Orthesenkomponente 30 zu erkennen, die über einen Gurt 60, der in einer den Oberschenkel umschließenden Manschette angeordnet ist, an dem Oberschenkel festlegbar ist. Unterhalb des Orthesenkniegelenkes 34 ist eine Unterschenkelmanschette 60 angeordnet, im Bereich des Orthesenknöchelgelenks 45 ist eine Knöchelmanschette 60 angeordnet, um den Unterschenkel an der Unterschenkelschiene 40 festzulegen. Über das Orthesenknöchelgelenk 45 ist das Fußteil 50 schwenkbar mit der Unterschenkelschiene 40 verbunden. Neben einer Dorsalextension oder Plantarflexion um das Orthesenknöchelgelenk 45 kann eine Verschwenkung des Fußteils 50 zusätzlich um eine im Wesentlichen waagerecht orientierte Schwenkachse erfolgen, die in der Sagittalebene orientiert ist.

Der Figur 8 ist zu entnehmen, dass mehrere Antriebe 70 der Orthese zugeordnet sind, die jeweils eine Flexion bzw. Extension der jeweiligen Orthesenkomponente ermöglichen. Ein Antrieb 70 ist zwischen dem Befestigungsabschnitt 33 und dem mittleren Abschnitt 32 angeordnet, um eine Flexion des Oberschenkels um die Schwenkachse 25 des Gelenks 22 zu ermöglichen. An der Oberschenkelschiene 31 ist darüber hinaus ein weiterer Antrieb 70 zur Flexion oder Extension der Unterschenkelschiene 40 relativ zu der Oberschenkelschiene 31 um das Orthesenkniegelenk 34 positioniert. Zur Flexion um die Knöchelgelenksachse ist an der Unterschenkelschiene 40 ein dritter Antrieb 70 angeordnet, so dass die wesentlichen Bewegungen des Gehens durch die Antriebe 70 eingeleitet oder unterstützt werden können.

Der Seitenansicht gemäß Figur 8 ist zu entnehmen, dass das erste Gelenk 21 und damit auch die erste Schwenkachse 24, die die Abduktion und Adduktion des Beines ermöglicht, auf einem gemeinsamen Höhenniveau mit dem gemeinsamen Punkt 100 oder dem virtuellen Hüftgelenk 100 liegt. Das zweite Drehgelenk 22, auf der Schwenkachse 25, das für die Extension und Flexion vordringlich zuständig ist, liegt unterhalb des gemeinsamen Punktes 100, also distal versetzt zu der ersten Schwenkachse 24, die Gelenkachse 25 kann aber auch auf der gleichen Höhe oder oberhalb des gemeinsamen Punktes 100 liegen. Das dritte Gelenk 23 liegt zusätzlich distal zu dem zweiten Drehgelenk 22 oder zur Schwenkachse 25 versetzt und damit unterhalb des Beckens, ungefähr auf der Mitte zwischen dem gemeinsamen Punkt 100 und dem Orthesenkniegelenk 34.

Figur 9 zeigt die Ausgestaltung der Orthese 10 mit einem um die Schwenkachse 25 flektierte Stellung der rechten unteren Extremität. Eine Verschwenkung findet um das fiktive Hüftgelenk 100 statt, also um den Punkt, in dem sich die drei Achsen der Gelenkeinrichtung 20 schneiden. Zur Bewirkung einer nahezu natürlichen Bewegung wird gleichzeitig das Orthesenkniegelenk 34 flektiert, so dass die Unterschenkelschiene 40 im Wesentlichen senkrecht orientiert verbleibt.

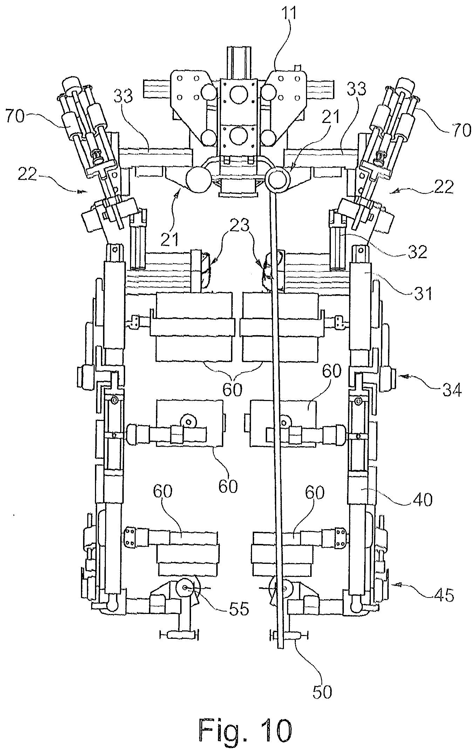

Figur 10 zeigt eine Ausgestaltung der Orthese in Gestalt einer Beinorthese in Frontalansicht. Neben der Befestigungseinrichtung 11 sind die ersten Gelenke 21 zur Ausführung einer Abduktion und Adduktion zwischen der Befestigungseinrichtung 11 und dem Befestigungsabschnitt 33 beiderseits einer Mittelschiene angeordnet. Die Antriebe 70 zur Bewirkung einer Verdrehung des zweiten Gelenkabschnittes 32 relativ zu dem Befestigungsabschnitt 33 um das zweite Gelenk 22 ist ebenso zu erkennen wie die fluchtende Ausrichtung der Schwenkachsen des ersten Gelenkes 21 mit dem dritten Gelenk 23, das überwiegend zur Innenrotation und Außenrotation der Beinorthese beiträgt.

Die Manschetten 60 mit den Gurten zur Festlegung der Orthese an der jeweiligen unteren Extremität sind ebenso zu erkennen wie die Orthesenkniegelenke 34, die Orthesenknöchelgelenke 45 sowie die Schwenkachse 55 zur Bewirkung eines Verschwenkens des Fußteiles 50 nach außen oder nach innen.

## Patentansprüche

1. Beinorthese (10) mit einer Befestigungseinrichtung (11) zur Festlegung der Beinorthese (10) an einem Rumpf (2) eines Orthesennutzers (1) und zumindest einer Gelenkeinrichtung (20), über die eine an einer Extremität (3) des Orthesennutzers (1) festlegbare erste Orthesenkomponente (30) relativ zu der Befestigungseinrichtung (11) verschwenkbar gelagert ist, wobei die Gelenkeinrichtung (20) zumindest drei Gelenke (21, 22, 23) aufweist, die jeweils zumindest eine Schwenkachse (24, 25, 28) aufweisen und die jeweils zumindest einen Schwenkachsen (24, 25, 28) sich in einem gemeinsamen Punkt (100) schneiden, **dadurch gekennzeichnet, dass** zumindest zwei der Schwenkachsen (24, 25, 28) nicht orthogonal zueinander ausgerichtet sind.

2. Beinorthese nach Anspruch 1, **dadurch gekennzeichnet, dass** der Gelenkeinrichtung (20) zumindest ein Antrieb oder Kraftspeicher zugeordnet ist.

3. Beinorthese nach Anspruch 1 oder 2, **dadurch gekennzeichnet, dass** die Freiheitsgrade einer Schwenkachse (25) zumindest eines Gelenkes (22) der Gelenkeinrichtung (20) die Freiheitsgrade der Schwenkachsen (24, 28) der übrigen Gelenke (21, 23) nicht beeinflussen.

4. Beinorthese nach einem der voranstehenden Ansprüche, **dadurch gekennzeichnet, dass** die Schwenkachsen (24, 25, 28) sich in dem Drehpunkt des Hüftgelenkes des Orthesennutzers (1) schneiden.

5. Beinorthese nach einem der voranstehenden Ansprüche, **dadurch gekennzeichnet, dass** die Befestigungseinrichtung (11) zur Festlegung der Beinorthese (10) an dem Becken, dem Bauchbereich oder der Taille und die erste Orthesenkomponente (30) als Oberschenkelschiene ausgebildet sind.

6. Beinorthese nach einem der voranstehenden Ansprüche, **dadurch gekennzeichnet, dass** die erste Orthesenkomponente (30) mehrere, gelenkig miteinander verbundene Abschnitte (31, 32, 33) aufweist.

7. Beinorthese nach einem der voranstehenden Ansprüche, **dadurch gekennzeichnet, dass** an der ersten Orthesenkomponente (30) zumindest eine weitere Orthesenkomponente (40, 50) gelenkig befestigt ist.

8. Beinorthese nach Anspruch 7, **dadurch gekennzeichnet, dass** die zumindest eine weitere Orthesenkomponente (40, 50) an der Extremität (3) festlegbar ausgebildet ist.

9. Beinorthese nach Anspruch 7 oder 8, **dadurch gekennzeichnet, dass** die Schwenkachsen (36, 47) der Gelenke (34, 45) zwischen den weiteren Orthesenkomponenten (40, 50) im angelegten Zustand der Beinorthese (10) mit Gelenkachsen (6, 7) der Extremität (3) fluchten.

10. Beinorthese nach einem der voranstehenden Ansprüche, **dadurch gekennzeichnet, dass** die Schwenkachsen (24, 25, 28) der Gelenke (21, 22, 23) der Gelenkeinrichtung (20) jeweils genau einen Freiheitsgrad aufweisen.

11. Beinorthese nach einem der voranstehenden Ansprüche, **dadurch gekennzeichnet, dass** der Schwenkbereich der Gelenke (21, 22, 23, 36, 45) einstellbar ist.

12. Beinorthese nach einem der voranstehenden Ansprüche, **dadurch gekennzeichnet, dass** eine Schwenkachse (25) eine Extension und Flexion der Hüfte ermöglicht und der Gelenkeinrichtung (20) eine um diese Schwenkachse (25) wirksamer Antrieb zugeordnet ist.

13. Beinorthese nach einem der voranstehenden Ansprüche, **dadurch gekennzeichnet, dass** bei einer angetriebenen Achse (25) keine Momente auf die anderen Achsen (24, 28) ausgeübt werden.

14. Beinorthese nach einem der voranstehenden Ansprüche, **dadurch gekennzeichnet, dass** Momente um eine nicht angetriebene Achse (25) oder mehrere nicht angetriebene Achse (24, 28) keinen Einfluss auf die anderen Achsen (24, 28) oder die andere Achse (25) haben.

## Claims

1. A leg orthosis (10) comprising a securing device (11) for fixing the leg orthosis (10) to a trunk of the body (2) of an orthosis user (1) and at least one articulation device (20), by way of which a first orthosis component (30), which can be fixed to an extremity (3) of the orthosis user (1), is mounted pivotably relative to the securing device (11), the articulation device (20) has at least three joints (21, 22, 23), which respectively have at least one pivot axis (24, 25, 28) and the respectively at least one pivot axes (24, 25, 28) intersect at a common point (100), **characterized in that** at least two of the pivot axes (24, 25, 28) are not aligned orthogonally in relation to one another.

2. The leg orthosis as claimed in claim 1, **characterized in that** the articulation device (20) is assigned at least one drive or energy store.

3. The leg orthosis as claimed in claim 2, **characterized in that** the degrees of freedom of a pivot axis (25) of at least one joint (22) of the articulation device (20) do not influence the degrees of freedom of the pivot axes (24, 28) of the other joints (21, 33).

4. The leg orthosis as claimed in one of the preceding claims, **characterized in that** the pivot axes (24, 25, 28) intersect at the point of rotation of the hip joint of the orthosis user (1).

5. The leg orthosis as claimed in one of the preceding claims, **characterized in that** the securing device (11) is designed for fixing the leg orthosis (10) to the pelvis, the area of the abdomen or the waist and the first orthosis component (30) is designed as an upper leg rail.

6. The leg orthosis as claimed in one of the preceding claims, **characterized in that** the first orthosis component (30) has a number of portions (31, 32, 33) that are connected to one another in an articulated manner.

7. The leg orthosis as claimed in one of the preceding claims, **characterized in that** at least one further orthosis component (40, 50) is secured in an articulated manner on the first orthosis component (30).

8. The leg orthosis as claimed in claim 7, **characterized in that** the at least one further orthosis component (40, 50) is formed such that it can be fixed to the extremity (3).

9. The leg orthosis as claimed in claim 7 or 8, **characterized in that** the pivot axes (36, 47) of the joints (34, 45) between the further orthosis components (40, 50) are in line with the joint axes (6, 7) of the extremity (3) when the leg orthosis (10) is in the fitted state.

10. The leg orthosis as claimed in one of the preceding claims, **characterized in that** the pivot axes (24, 25, 28) of the joints (21, 22, 23) of the articulation device (20) have in each case precisely one degree of freedom.

11. The leg orthosis as claimed in one of the preceding claims, **characterized in that** the pivoting range of the joints (21, 22, 23, 36, 45) is adjustable.

12. The leg orthosis as claimed in one of the preceding claims, **characterized in that** a pivot axis (25) enables an extension and flexion of the hip and the articulation device (20) is assigned a drive that is effective about this pivot axis (25).

13. The leg orthosis as claimed in one of the preceding claims, **characterized in that**, when one axis (25) is driven, no moments are exerted on the other axes (24, 28).

14. The leg orthosis as claimed in one of the preceding claims, **characterized in that** moments about a non-driven axis (25) or a number of non-driven axes (24, 28) have no influence on the other axes (24, 28) or the other axis (25).

## Revendications

1. Orthèse de jambe (10) comportant un moyen de fixation (11) pour immobiliser l'orthèse de jambe (10) sur un tronc (2) d'un utilisateur d'orthèse (1) et au moins un moyen d'articulation (20) via lequel un premier composant d'orthèse (30) immobilisable sur une extrémité (3) de l'utilisateur d'orthèse (1) est monté mobile en basculement par rapport au moyen de fixation (11), le moyen d'articulation (20) présentant au moins trois articulations (21, 22, 23) qui présentent chacune au moins un axe de basculement (24, 25, 28), et lesdits axes de basculement (24, 25, 28) se recoupent chacun en un point commun (100),
**caractérisée en ce que**
au moins deux des axes de basculement (24, 25, 28) ne sont pas orientés orthogonalement les uns par rapport aux autres.

2. Orthèse de jambe selon la revendication 1,
**caractérisée en ce que**
au moins un entraînement ou un accumulateur de force est associé au moyen d'articulation (20).

3. Orthèse de jambe selon la revendication 1 ou 2,
**caractérisée en ce que**
les degrés de liberté d'un axe de basculement (25) d'au moins une articulation (22) du moyen d'articulation (20) n'influencent pas les degrés de liberté des axes de basculement (24, 28) des autres articulation (21, 23).

4. Orthèse de jambe selon l'une des revendications précédentes,
**caractérisée en ce que**
les axes de basculement (24, 25, 28) se recoupent au point de rotation de l'articulation de la hanche de l'utilisateur d'orthèse (1).

5. Orthèse de jambe selon l'une des revendications précédentes,
**caractérisée en ce que**
le moyen de fixation (11) pour immobiliser l'orthèse de jambe (10) sur le bassin, sur la zone ventrale ou sur la taille et le premier composant d'orthèse (30) sont réalisés sous la forme d'une attelle de fémur.

6. Orthèse de jambe selon l'une des revendications précédentes,
**caractérisée en ce que**
le premier composant d'orthèse (30) comprend plusieurs portions (31, 32, 33) reliées en articulation les unes aux autres.

7. Orthèse de jambe selon l'une des revendications précédentes,
**caractérisée en ce que**
au moins un autre composant d'orthèse (40, 50) est fixé en articulation au premier composant d'orthèse (30).

8. Orthèse de jambe selon la revendication 7,
**caractérisée en ce que**
ledit au moins un autre composant d'orthèse (40, 50) est réalisé de manière à pouvoir être immobilisé sur l'extrémité (3).

9. Orthèse de jambe selon la revendication 7 ou 8,
**caractérisée en ce que**
les axes de basculement (36, 47) des articulations (34, 45) entre les autres composants d'orthèse (40, 50) sont en alignement avec des axes d'articulation (6, 7) de l'extrémité (3), dans l'état appliqué de l'orthèse de jambe (10).

10. Orthèse de jambe selon l'une des revendications précédentes,
**caractérisée en ce que**
les axes de basculement (24, 25, 28) des articulations (21, 22, 23) du moyen d'articulation (20) présentent chacun précisément un degré de liberté.

11. Orthèse de jambe selon l'une des revendications précédentes,
**caractérisée en ce que**
la plage d'articulation des articulations (21, 22, 23, 36, 45) est réglable.

12. Orthèse de jambe selon l'une des revendications précédentes,
**caractérisée en ce que**
un axe de basculement (25) permet une extension et une flexion de la hanche, et un entraînement agissant autour de cet axe de basculement (25) est associé au moyen d'articulation (20).

13. Orthèse de jambe selon l'une des revendications précédentes,
**caractérisée en ce que**
l'axe (25) étant entraîné, aucun couple n'est exercé sur les autres axes (24, 28).

14. Orthèse de jambe selon l'une des revendications précédentes,
**caractérisée en ce que**
des couples autour d'un axe (25) non entraîné ou autour de plusieurs axes (24, 28) non entraînés n'ont pas d'influence sur les autres axes (24, 28) ou sur l'autre axe (25).
